(19) Europäisches Patentamt
European Patent Office
Office européen des brevets

(11) Publication number: **0 136 464**
**B1**

(12) # EUROPEAN PATENT SPECIFICATION

(45) Date of publication of patent specification: **26.10.88**

(51) Int. Cl.$^4$: **A 61 K 31/70,** A 61 K 9/36, A 61 K 9/52

(21) Application number: **84109110.1**

(22) Date of filing: **01.08.84**

(54) **Therapeutic compositions for oral use containing stable S-Adenosy1-L-Methionine salts.**

(30) Priority: **24.08.83 IT 2262183**

(43) Date of publication of application:
**10.04.85 Bulletin 85/15**

(45) Publication of the grant of the patent:
**26.10.88 Bulletin 88/43**

(84) Designated Contracting States:
**AT BE CH DE FR GB LI LU NL SE**

(56) References cited:
**EP-A-0 063 014**
**EP-A-0 073 376**
**DE-A-2 745 705**
**US-A-4 057 686**

(73) Proprietor: **BIORESEARCH S.p.A.**
**Località Roggia Pirola**
**I-20060 Liscate Milano (IT)**

(72) Inventor: **Gennari, Federico**
**Via Leonardo da Vinci 52**
**Truccazzano Milano (IT)**
Inventor: **Aspesi, Pier Luigi**
**Via Cosseria, 4**
**Milano (IT)**

(74) Representative: **Gervasi, Gemma, Dr. et al**
**Studio Brevetti e Marchi**
**NOTABARTOLO & GERVASI**
**33, Viale Bianca Maria**
**1-20122 Milano (IT)**

**EP 0 136 464 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new therapeutic compositions for oral use, containing stable S-adenosyl-L-methionine salts, a polyhydroxilated organic compound and an external coating based on a gastro-resistant polymer.

Sulpho-adenosyl-L-methionine (SAMe) (I) is known to be the main biological donor of methyl groups.

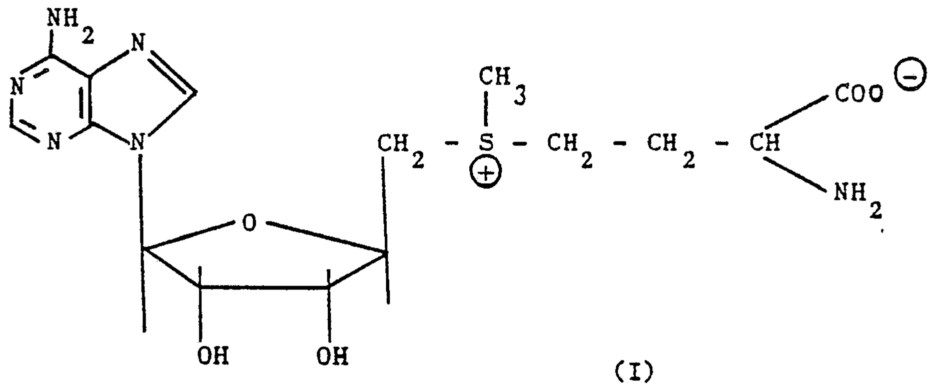

(I)

This special characteristic has made it a subject of considerable interest, initially from the biochemical aspect and subsequently because of its possible therapeutic applications.

However, its practical use in human therapy was impossible for many years because of two apparently insoluble problems, namely the instability of SAMe at ambient temperature and the impossibility of producing and purifying it by methods which can be implemented on an industrial scale.

The aforesaid problems have been solved by the present applicant, who has filed numerous patents (for example USA patents 3,893,999, 3,954,726, 4,057,686 and European patent application 73.376) which aim at preparing stable SAMe salts by industrial methods.

Stable SAMe salts have been administered up to the present time only by injection for two basic reasons:

— the strong acidity of these salts, which makes them aggressive towards the gastric mucous membranes,

— the proved ineffectiveness of all SAMe salts when administered orally.

It has now been found, and forms the subject matter of the present invention, that although stable SAMe salts are practically unabsorbed when administered orally, they are absorbed to a considerable extent if administered directly into the intestine.

This is entirely surprising, in that nothing in the structure of SAMe salts could lead to the prediction of such a different absorption capacity at different levels of the gastro-enteric tract, and in particular in view of their proved ineffectiveness when administered orally.

As a consequence of the present discovery, new therapeutic compositions containing one or more stable SAMe salts as active principle have been prepared, which are gastro-resistant and entero-soluble, and are thus able to allow direct administration of the active principle into the intestine, even though administered orally.

Anti-inflammatory and analgesic activity has also been discovered for SAMe salts when administered orally, such activity never having previously been predicted for these salts.

It is however clear that the new compositions for oral administration according to the invention are not limited to using SAMe salts as anti-inflammatory or analgesic agents, and instead can be utilised for administering said salts for any of the known therapeutic purposes by suitably varying the dose.

In order to demonstrate the different absorption of stable SAMe salts when administered gastrically and intestinally, an entire series of tests has been carried out, the results of which are given in the following Table 1.

The values given relate to tests carried out with SAMe-tri-p. toluenesulphonate, but substantially identical results were obtained with other known SAMe salts.

The drug concentrations in the portal or aortic plasma were evaluated as the absorption parameters.

The experiment was carried out on 72 male Sprague-Dawley rats of weight 210—260 g, which had fasted since the previous evening.

The animals, anesthetised with ether, were operated on in order to insert a capsule into the proximal jejunum. The incision in the intestinal wall was closed by ligature, and the animals were sutured.

The treatment scheme was as follows:

Intestinal administration:

A capsule containing a weighed SAMe quantity corresponding to a dose of 32 mg/kg of body weight was inserted into the intestine, and 5 ml of $H_2O$/kg were administered by means of a gastric probe.

Oral administration:

An empty capsule was inserted into the intestine, and 32 mg of SAMe/kg in aqueous solution (5 ml/kg) were administered by means of a gastric probe.

Controls:

An empty capsule was inserted into the intestine, and 5 ml of $H_2O$/kg of body weight were administered by means of a gastric probe.

After 10, 20, 40, 60, 90, 120, 180 and 240 minutes from administration, 3 animals were sacrificed at a time in order to withdraw portal and aortic blood.

The SAMe concentrations in the plasma were determined by the Baldessarini and Kopin method (J. Neuroch. 13: 769, 1966).

The values represent the mean ± standard error of 3 observations, reduced by the values found for the controls.

TABLE 1

SAMe concentration in the plasma (mcg/ml).

| Time | Intestinal treatment | | Oral treatment | |
|---|---|---|---|---|
| (min) | Portal | Aortic | Portal | Aortic |
| 10 | 25.5 | 13.1 | 2.93 | 1.1 |
| 20 | 9.39 | 8.63 | 0.87 | 0.78 |
| 40 | 2.99 | 4.39 | 0.84 | 0.72 |
| 60 | 2.44 | 1.87 | 0.61 | 0.70 |
| 90 | 1.54 | 1.28 | 0.54 | 0.68 |
| 120 | 0.77 | 0.88 | 0.52 | 0.68 |
| 180 | 0.77 | 0.69 | 0.52 | 0.66 |
| 240 | 0.64 | 0.53 | 0.46 | 0.64 |

These values show that although good time-persistent SAMe concentrations in the plasma are obtained when SAMe salts are directly administered into the intestine, in the case of normal oral administration the gastro-enteric absorption is a minimum and thus the plasma values are very low.

The same conclusions are drawn when evaluating the antiphlogistic and analgesic action of stable SAMe salts when administered orally and intestinally.

In the experiments carried out on animals which received the SAMe orally, the drug was dissolved in physiological solution and administered by gastric probe in a volume of 0.5 ml/100 g of body weight. An equal volume of physiological solution was administered to the controls.

In the experiments in which SAMe was administered intraduodenally, the animals anesthetised with ether were subjected to laparotomy to allow the insertion into the duodenum of gelatin capsules (size 4) containing the product.

After inserting the capsule, the duodenum was sutured carefully so as not to traumatise the tissue and was then returned to its seat, the muscular and cutaneous layers then being re-composed and sutured.

The control animals received an empty gelatin capsule by the same procedure.

15 minutes after the operation, the animals showed complete recovery.

the antiphlogistic and analgesic activity of SAMe were evaluated by the following tests:

A. *Anti-inflammatory activity*

1) Edema induced by carrageen in the rat.

Male Sprague-Dawley rats of about 170 g weight were used, having fasted since the evening preceding the experiment. Edema was induced by carrageen using the method described by Winter et al. (Proc. Soc. Exp. Biol. Med. 111, 544 (1962)).

The SAMe was administered orally one hour before the carrageen at doses of 25 and 50 mg/kg, and intraduodenally at doses of 25 and 50 mg/kg 80 minutes before the phlogogenic agent.

Indomethacin was used as the comparison drug, and was administered orally at a dose of 9 mg/kg before the carrageen.

Tables 2 and 3 show the results obtained by oral and intraduodenal administration of SAMe on edema induced by carrageen. The results show that:

— oral administration of the product at doses of 25 and 50 mg/kg is not able to significantly modify the edema development, which in the treated animals is practically identical to that of the control animals indicated in Table 2 by "physiological solution";

— in the case of intraduodenal SAMe administration, the results shown in Table 3 indicate an effect difference between the doses of 25 and 50 mg/kg, and a statistically significant edema inhibition at the 50 mg/kg dose.

TABLE 2 - Effect of orally administered SAMe on edema induced by carrageen in the rat

| Treatment | Dose (mg/kg) | Percentage increase in paw volume at various times after carrageen administration | | | | |
|---|---|---|---|---|---|---|
| | | 1 h | 2 h | 3 h | 4 h | 5 h |
| Physiological soln. | - | 29.9 | 34.3 | 51.4 | 54.4 | 60.4 |
| SAMe | 25 | 33.6 | 39.0 | 46.9 | 52.7 | 53.4 |
| SAMe | 50 | 27.0 | 32.7 | 45.8 | 53.2 | 56.7 |

The values are the mean ± S.E. of 12 animals per group

TABLE 3 - Anti-inflammatory activity of SAMe administered into the duodenum on edema induced by carrageen in the rat

| Treatment | Dose (mg/kg) | Percentage increase in paw volume at various times after carrageen administration 1 h | 2 h | 3 h | 4 h |
|---|---|---|---|---|---|
| Empty capsule | - | 20.0 | 29.0 | 40.0 | 46.9 |
| SAMe | 25 | 16.1 (19.5) | 23.8 (18) | 32.2 (19) | 39.5 (16) |
| SAMe | 50 | 13.2 | 17.4 (40) | 24.1 (40) | 31.8 (32) |
| Indomethacin | 9 | 13.4 (33) | 21.6 (25) | 35.3 (12) | 40.1 (14) |

The indomethacin was administered orally to rats which received one empty capsule into the duodenum

The values represent the mean ± S.E. of 12 animals. The percentage inhibition with respect to the controls is given in parentheses.

B. *Analgesic activity*

1) Reaction to heat, tail flick test in the rat.

The analgesic activity of SAMe was evaluated in male Sprague-Dawley rats of about 190 g weight by the method of D'Amour and Smith (J. Pharmacol. Exp Theor. 71, 74 (1941)).

The SAMe was administered either orally or in gelatin capsules into the duodenum in doses of 50 and 100 mg/kg.

Morphine was used as the comparison drug and was administered under the cutis at a dose of 5 mg/kg.

The analgesic activity was determined 30, 60 and 120 minutes after administration of the drugs.

Tables 4 and 5 show the results of the tail flick test in the rat.

The results represent the mean ± S.E. for the number of animals indicated in parentheses. The morphine was administered under the cutis to rats which received one empty capsule into the duodenum.

The data obtained at the 30 minute determination for treatment with SAMe were analysed by randomised block variance analysis. This anslysis shows a highly significant difference ($P < 0.01$) between the animals treated with SAMe and the controls.

Furthermore, the Dunnet test indicated that 100 mg/kg of SAMe increase the reaction time to heat in a highly significant manner ($P < 0.01$) in the case of treated animals, with respect to the controls.

The animals treated with morphine showed reaction times significantly greater than those of the controls at all observation times.

From the results shown it is apparent that oral SAMe administration is not able to modify the reaction time towards heat with respect to the controls.

Good analgesic activity is however present after intraduodenal SAMe administration. The statistical analysis carried out on the reaction times determined 30 minutes after treatment indicates a highly significant difference ($P < 0.01$) between the animals treated with SAMe at a dose of 100 mg/kg and the controls.

Morphine exerts a powerful analgesic activity ($P < 0.01$) with respect to the controls at all observation times.

TABLE 4 - Analgesic activity of SAMe administered orally in the tail flick test in the rat

| Treatment | Dose (mg/kg) | Reaction time in minutes at various times after treatment | | | |
|---|---|---|---|---|---|
| | | $T_0$ | 30 min | 60 min | 120 min |
| Physiological solution | - | 4.54 | 4.94 | 4.35 | 4.28 |
| SAMe | 50 | 4.60 | 5.04 | 4.09 | 4.20 |
| SAMe | 100 | 4.41 | 4.46 | 4.73 | 4.25 |

The data represent the mean ± S.E. of 13 animals per group

TABLE 5 - Analgesic activity of SAMe administered into the duodenum in the tail flick test in the rat

| Treatment | Dose (mg/kg) | Reaction time in minutes at various times after treatment | | | |
|---|---|---|---|---|---|
| | | $T_0$ | 30 min | 60 min | 120 min |
| Empty capsule | - | 5.2 | 6.6 | 7.5 | 8.4 |
| SAMe (14) | 50 | 4.8 | 8.5 | 8.2 | 8.3 |
| SAMe (14) | 100 | 5.0 | 10.2 | 9.6 | 8.5 |
| Morphine (13) | 5 | 4.7 | 19.8 | 18.9 | 14.1 |

From the results obtained in the experiments carried out in order to identify the anti-inflammatory and analgesic activity of SAMe, of which the two described tests are only an example, it can be concluded that only intraduodenal administration of the product is able to inhibit the development of the inflammatory process and to reduce heat sensitivity, i.e. induce an analgesic effect.

In order to enable the invention to be implemented, i.e. in order to administer therapeutic compositions based on SAMe salts directly into the intestine by an oral path, new therapeutic compositions have been formed with the following essential characteristics:

— gastro-resistance protracted for at least two hours with no release of active principle

— very rapid disgregation in the enteric juices, so as to provide maximum availability of the drug in the shortest possible time.

These objects are attained by using an entero-soluble excipient in a quantity of between 50 and 400 mg per tablet, chosen from the poly-hydroxylated organic compound group, essentially composed of saccharides, oligosaccharides and polysaccharides such as sugars, starches and cellulose. Other solid excipients may be added to this essential excipient, but must satisfy the following requirements:

— they must not contain more than 2% of free moisture

— they must preferably exercise an absorbing action towards external moisture

— they must the of very fine particle size and possess good compressibility characteristics.

Examples of formulations satisfying the requirements of the present invention are given by way of non-limitative illustration hereinafter:

A)

| | |
|---|---|
| SAMe sulphate-p.toluenesulfonate | 384 mg |
| Starch | 230 mg |
| Silica | 192 mg |
| Magnesium stearate | 10 mg |

B)

| | |
|---|---|
| SAMe sulphate-p.toluenesulfonate | 384 mg |
| Mannite | 166 mg |
| Silica | 40 mg |
| Magnesium stearate | 10 mg |

C)

| | |
|---|---|
| SAMe sulphate-p.toluenesulfonate | 384 mg |
| Mannite | 179 mg |
| Magnesium stearate | 6 mg |

D)

| | |
|---|---|
| SAMe sulphate-p.toluenesulfonate | 384 mg |
| Silica | 90 mg |
| Starch | 77 mg |
| Magnesium stearate | 7 mg |
| Talc | 6 mg |

E)

| | |
|---|---|
| SAMe sulphate-p.toluenesulfonate | 384 mg |
| Silica | 60 mg |
| Starch | 77 mg |
| Avicel | 30 mg |
| Magnesium stearate | 10 mg |

In order to attain the object of the present invention, it is also essential to coat the compositions comprising the stable SAMe salt as active principle with a gastro-resistant and entero-soluble coating acting in the manner and with the times heretofore indicated.

Such a coating is formed for example from a suitable cellulose ester, a polyacrylate, a polymethacrylate, a polysiloxane or their mixtures, applied in a quantity of between 3 and 5 mg per tablet and mixed with the usual plasticisers and inert fillers.

Some illustrative but non-limiting examples of such coatings are as follows:

A)

| | |
|---|---|
| Cellulose acetophthalate | 20 mg |
| Diethylphthalate | 6.6 mg |
| Silicone | 2.3 mg |

B)

| | |
|---|---|
| Cellulose acetophthalate | 35 mg |
| Diethylphthalate | 9 mg |
| Silicone | 6 mg |

C)

| | |
|---|---|
| Methacrylic acid/methyl methacrylate polymer | 20 mg |
| Butyl phthalate | 2 mg |
| 50%/50% mixture of talc and magnesium stearate | 5 mg |

D)

| | |
|---|---|
| Methacrylic acid/methyl methacrylate polymer | 15 mg |
| Polyethylene glycol | 1 mg |
| 50%/50% mixture of talc and magnesium stearate | 5 mg |

**Claims**

1. Therapeutic compositions for oral use containing stable S-adenosyl-L-methionine (SAMe) salts, a polyhydroxylated organic compound chosen from disaccharides, oligosaccharides and polysaccharides and an external coating based on a gastro-resistant polymer chosen from cellulose esters, polyacrylates, polymethacrylates, polysiloxanes and their mixtures.

2. Use of stable S-adenosyl-L-methionine (SAMe) salts for the manufacture of a gastro-resistant and enterosoluble pharmaceutical composition for therapeutic application as anti-inflammatory and analgesic.

**Patentansprüche**

1. Therapeutische Zusammensetzungen zur oralen Verwendung, enthaltend stabile S-Adenosyl-L-methionin (SAMe)-salze, eine polyhydroxylierte organische Verbindung ausgewählt aus Disacchariden, Oligosacchariden und Polysacchariden und einen Außenüberzug auf Basis eines magenreistenten Polymers ausgewählt aus Zelluloseestern, Polyacrylaten, Polymethacrylaten, Polysiloxanen und deren Mischungen.

2. Verwendung von stabilen S-Adenosyl-L-methionin (SAMe)-salzen für die Herstellung einer magenresisten und darmlöslichen pharmazeutischen Zusammensetzung für die therapeutische Anwendung als entzündungshemmendes und analgetisches Mittel.

**Revendications**

1. Compositions thérapeutiques pour l'utilisation orale contenant des sels de S-adénosyl-L-méthionine (SAMe) stables, un composé organique polyhydroxylé chloisi parmi des disaccharides, des oligosaccharides et des polysaccharides et un enrobage externe basé sur un polymère gastro-résistant choisi parmi des esters cellulosiques, des polyacrylates, des polyméthacrylates, des polysiloxanes et leurs mélanges.

2. Utilisation de sels de S-adénosyl-L-méthionine (SAMe) stables pour la fabrication d'une composition pharmaceutique gasro-résistante et entéro-soluble pour l'application thérapeutique comme anti-inflammatoire et analgésique.